# EUROPEAN PATENT SPECIFICATION

(11) **EP 1 243 298 B1**
(45) Date of publication and mention of the grant of the patent: **14.01.2009**
(21) Application number: 00962966.8
(22) Date of filing: 29.09.2000
(51) Int. Cl.: B01D 15/00, A61M 1/36, C02F 1/28, B01D 24/04, B01D 27/00, B01D 27/08

(54) **FILLING OUT-FLOW PREVENTING FILTER AND ABSORBER ATTACHED WITH IT**
ABSORBER MIT DARAN BEFESTIGTEM FILTER ZUR VERHINDERUNG VON ÜBERLAUF
FILTRE ANTI-DEBORDEMENT ET ABSORBEUR SUR LEQUEL IL EST FIXE

(30) Priority: 02.11.1999 JP 31210499
(43) Date of publication of application: 25.09.2002
(73) Proprietor: KANEKA CORPORATION, Osaka-shi, Osaka 530-8288 (JP)
(72) Inventor: ITO, Shinji, Osaka 566-0072 (JP); NANKO, Toshiki, Kobe-shi, Hyogo 653-0841 (JP); FURUYOSHI, Shigeo, Kobe-shi, Hyogo 654-0152 (JP)
(74) Representative: Gille Hrabal Struck Neidlein Prop Roos
(86) International application number: PCT/JP2000/006754
(87) International publication number: WO 2001/032286

(56) References cited:
- EP-A- 0 800 862
- GB-A- 2 233 246
- JP-A- 9 266 948
- JP-A- 63 020 089
- JP-U- 60 049 905
- US-A- 4 202 775
- US-A- 4 358 376

## Description

### Technical Field

This invention relates to an adsorber comprising a housing, a liquid port having a liquid inlet, a liquid port having a liquid outlet, a filling spillage preventing filter provided on the entrance and exit sides or only on the exit side, preferably on both the entrance and exit sides, and an adsorbing unit packed between the filters. It is particularly preferred to use the adsorber in medical applications.

### Background Art

Adsorbers comprise a housing, liquid ports at opposite ends of the housing which respectively have a liquid inlet and a liquid outlet, and a filling spillage preventing filter (hereunder referred to as a filter) that is provided between the housing and the liquid port on the exit side or on each of the entrance and exit sides in order to pack and retain the adsorbing unit within the housing. The housing is joined to the liquid ports as by ultrasonic welding or high-frequency welding; the filters are fitted into the liquid ports and are each fixed by being pressed between the housing and the liquid port. The filter used in this construction consists of a fabric mesh portion that allows the adsorbing unit to be retained within the housing and a ring portion for reinforcing the mesh portion. The housing and the liquid ports are molded with hard resins such as polycarbonates, polypropylenes, polystyrenes, etc. and the ring portion of the filter is also molded with hard resins such as polycarbonates, polypropylenes, polystyrenes, etc. If there is a small clearance between the filter and the housing or between the filter and each of the liquid ports, the liquid may leak into the gap between the filter and each liquid port while the adsorber is in service and after its service, the liquid remains between a lateral side of the filter and each liquid port. Conventionally, this problem has been solved by inserting a thin packing, typically made of silicone rubber, into the clearance between each filter and the liquid port and pressed between an end face of the filter and an end face of the housing, as well as between an end face of the filter and the inner surface of each liquid port so that no liquid will leak into the gap between the filter and the liquid port.

In the conventional method, if no packing is used, the liquid leaks into the gap between the filter and the liquid port; the use of a packing increases the number of constituent parts of the adsorber and, in addition, if the housing and the ring portion of the filter are small in wall thickness, the packing may potentially be displaced when the housing is welded or otherwise joined to the liquid ports. Furthermore, if the end faces of the housing, the inner surface of each liquid port and their surfaces in contact with the filters have flaws and molding asperities, no uniform pressing can be realized at the end faces of the housing or on the inner surfaces of the liquid ports; therefore, even if a packing is used, a gap occurs between the filter and an end face of the housing or between the filter and the inner surface of the liquid port and the liquid will seep into the gap between the filter and the liquid port. If the adsorber is used in sanitary applications, the gap provides a site for microbial growth, becomes inaccessible by cleaning devices or otherwise causes adverse effects such as by impairing product safety. If the adsorber is used in medical applications, namely, as a medical adsorber, a body fluid may leak into the space between the filter and the liquid port during service of the medical adsorber and, after the treatment, the body fluid may remain in the clearance between the filter and an end of the housing or between the filter and the inner surface of the liquid port, thus impairing the external appearance of the device. JP 63-20089 described a device for partying wafer.

### Disclosure of the Invention

The invention also relates to an adsorber as set forth in the claims.

### Brief Description of the Drawings

Fig. 1 is a sectional view of a medical adsorber according to an example of the invention;
Fig. 2 is a partial enlarged section of a housing fitted with a liquid port that is equipped with the filling spillage preventing filter in an example of the conventional medical adsorber, provided that the ring portion of the filling spillage preventing filter is formed of a hard resin;
Fig. 3 is a partial enlarged section of a housing fitted with a liquid port that is equipped with the filling spillage preventing filter in a medical adsorber according to an example of the present invention, provided that the ring portion of the filling spillage preventing filter is formed of a soft resin;
Fig. 4 is a partial enlarged view of a ridge projecting from the bottom of an annular recess in the port portion;

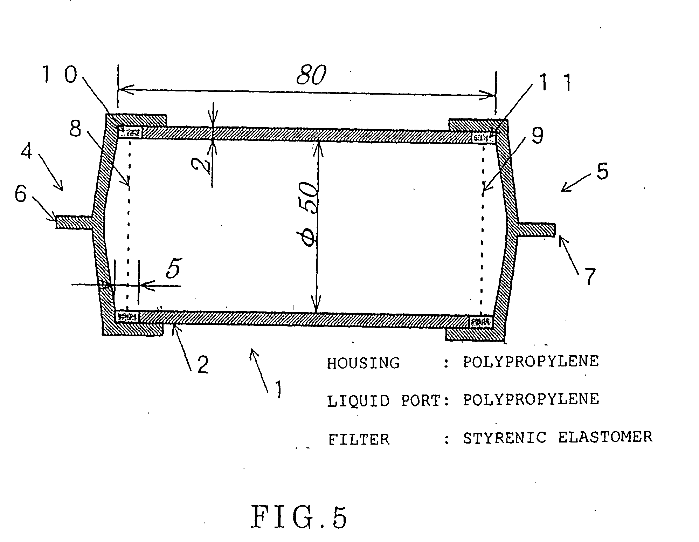

Fig. 5 is a diagram showing specific example 1 of the invention, in which the housing and the liquid ports are formed of polypropylene and the ring portion of the filters are formed of a styrenic elastomer;

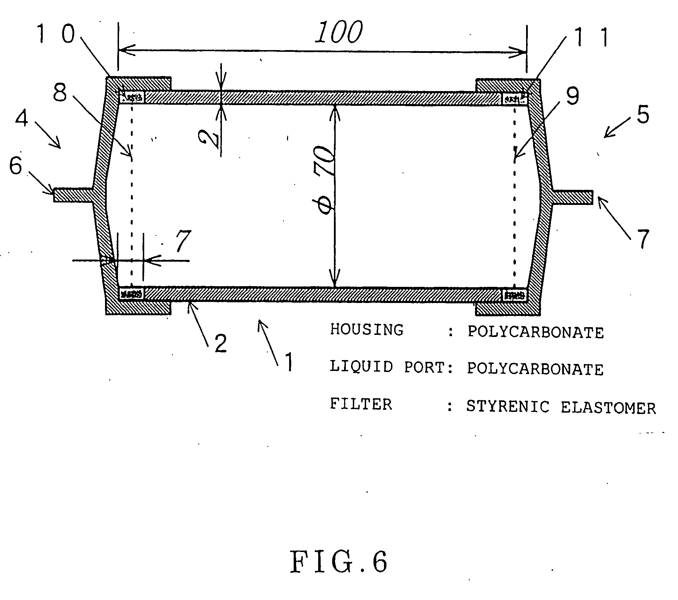

Fig. 6 is a diagram showing specific example 2 of the invention, in which the housing and the liquid ports are formed of polycarbonate and the ring portion of the filters are formed of a styrenic elastomer; and
Fig. 7 is a partial enlarged section of the ring portion, as indicated by 11 in Fig. 1 or 10 in Fig. 3, of the filling spillage preventing filter of the invention which uses a hard material in the portion which reinforces the periphery of the mesh portion, with a soft material being used in the surface of the ring portion and a hard material used in its interior with a view to bringing about the intended advantages of the invention.

In the drawings, numeral 1 designates the medical adsorber, 2 its housing, 3 the adsorbent, 4 one liquid port, 5 the other liquid port, 6 the liquid inlet, 7 the liquid outlet, 8 the mesh portion of one filling spillage preventing filter, 9 the mesh portion of the other filling spillage preventing filter, 10 the ring portion of one filling spillage preventing filter (which is made of a soft resin), 11 the ring portion of the other filling spillage preventing filter (which is made of a soft resin), 12 the ring portion of a filtering spillage preventing filter (which is made of a hard resin), 13 an annular rib, 14 a ridge, 15 a joint, 16 a hard material for reinforcing the periphery of the mesh portion, 17 an elastic soft resin, and 18 the mesh portion.

### Best Mode for Carrying Out the Invention

The invention has as its object providing an adsorber comprising a housing, filters fitted into liquid ports, and an adsorbing unit packed between the filters, characterized in that the number of constituent parts of the adsorber is not increased but the pathways and gaps through which the liquid will seep to the lateral sides of the filters are eliminated to ensure that the liquid will not leak into the clearance between each filter and a lateral side of the liquid port.

The stated object can be attained by an adsorber comprising a housing, an adsorbing unit comprising a multiple of adsorbents packed within said housing, a liquid port on the entrance side that has a liquid inlet and which is fitted in an end portion of said housing and a liquid port on the exit side that has a liquid outlet and which is fitted in the other end portion of said housing, characterized in that a filter composed of a structure having a mesh portion for retaining the adsorbing unit within the housing and an elastic soft resin portion is fixed pressed between the inner surface of each of said liquid ports and an end face of the housing.

By providing the above-described filter on the liquid port on the exit side or on both the entrance and exit sides, the adsorbing unit can be packed and retained within the housing. Each filter has a fabric mesh portion for retaining the adsorbing unit within the housing and a ring portion for reinforcing the periphery of said mesh portion; said ring portion may solely be formed of an elastic soft resin or, alternatively, only that part of said ring portion which contacts the inner surfaces of the housing and the liquid port may be formed of an elastic soft resin such that the periphery of said mesh portion is reinforced by a hard material whereas the surface of said hard material is covered with an elastic soft resin.

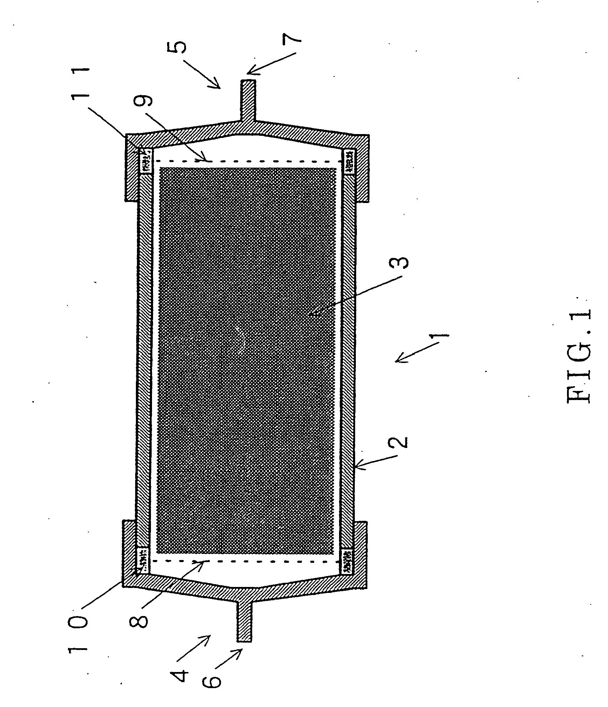

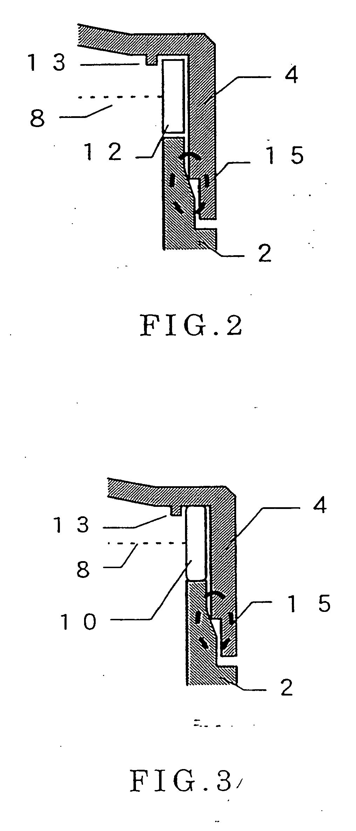

The adsorber of the invention is described below with reference to the examples shown in the drawings. Fig. 1 is a sectional view of an example of the adsorber of the invention; Fig. 2 is a partial enlarged section of a housing fitted with a liquid port equipped with a hard resin filter in an example of the conventional adsorber; and Fig. 3 is a partial enlarged section of a housing fitted with a liquid port equipped with a soft resin filter in an example of the adsorber of the invention.

As shown in Fig. 1, the adsorber of the example under consideration comprises a housing, an adsorbing unit comprising a multiple of adsorbents packed within said housing, a liquid port on the entrance side that has a liquid inlet and which is fitted in an end portion of said housing and a liquid port on the exit side that has a liquid outlet and which is fitted in the other end portion of said housing and it is characterized in that a filter composed of a structure having a mesh portion for retaining the adsorbing unit within the housing and an elastic soft resin portion is fixed pressed between the inner surface of each of said liquid ports and an end face of the housing.

By providing the above-described filter on the liquid port on the exit side or on both the entrance and exit sides, the adsorbing unit can be packed and retained within the housing. Each filter has a fabric mesh portion for retaining the adsorbing unit within the housing and a ring portion for reinforcing the periphery of said mesh portion; said ring portion may solely be formed of an elastic soft resin or, alternatively, only that part of said ring portion which contacts the inner surfaces of the housing and the liquid port may be formed of an elastic soft resin such that the periphery of said mesh portion is reinforced by a hard material whereas the surface of said hard material is covered with an elastic soft resin. The structural features of the adsorber are individually described below.

The housing of the adsorber of the example under consideration is a tubular molding and the adsorbing unit for processing the liquid is accommodated in the space defined by the filters and the housing. This adsorbing unit is packed within the housing in a liquid-tight manner.

The housing is formed of thermoplastic resins such as polycarbonates and polystyrenes or polyolefins such as polypropylene and polyethylene. It is of a tubular form, preferably of a tubular form having a circular cross section in order to provide a uniform flow through the adsorber, more preferably, the housing is in a cylindrical shape.

The liquid processing adsorbent may be built with any material and it can be used without particular limitations on shape and size. If the adsorber under consideration is to be used in medical applications, namely, if it is a medical adsorber, any building materials useful as medical materials can be used without particular limitations; any shapes and sizes will do if they can be accommodated within the housing but considering the ease of liquid passage, the preferred particle size is 10 µm - 2 mm and spherical particles with sizes of 10 - 600 µm are more preferred.

The liquid to be packed in the housing together with the adsorbing unit has no particular limitations on its properties or composition, for example as long as it fits the characteristics of the adsorbing unit. If the adsorber under consideration is to be used in medical applications, namely, if it is a medical adsorber, liquids that do no harm to the human body (e.g. physiological saline and sterile water) can be packed for use.

The liquid port is welded or otherwise joined to both end portions of the housing. Specifically, the liquid port has a joint to the housing as shown in Figs. 2 and 3, and this joint is securely fused to the housing by application of high-frequency waves or ultrasonic waves, for example.

The liquid ports are formed of thermoplastic resins such as polycarbonates and polystyrenes or polyolefins such as polypropylene and polyethylene.

The ring portion of the filter of the invention is characteristically formed of an elastic soft material; this is a material which, when pressed with an object, deforms in conformity with the object's shape developing the force of repulsion and which, upon removal of the object, restores its initial shape. In terms of resin's hardness, such elastic soft material has preferably values of no more than about JIS-A 120 degrees as determined by the method of JIS K 6301; in order to ensure that the ring portion has adequate rigidity even if it is solely made of a single material, hardness values of about JIS-A 60 - 100 degrees are preferred. The elastic soft material to be used in said ring portion is selected from among silicone rubbers, polyurethane rubbers, isoprene rubbers, vulcanized rubbers, butadiene rubbers, ethylene-propylene rubbers, polyolefin elastomers and styrenic elastomers. Among these materials, polyolefinic elastomers, styrenic elastomers and silicone rubbers are preferred.

If the filters require even more rigidity, one may design a structure in which the above-mentioned materials form the surface of the ring portion. To be more specific, only those parts of the filters that will contact the inner surfaces of the liquid ports and the end faces of the housing may be formed of the elastic soft material, with a hard material being used in the part that reinforces the periphery of the mesh portion. Any hard materials can be used without particular limitations on their composition as long as they have higher material hardness than the elastic soft resin used in the surface of the ring portion; preferably, thermoplastic resins such as polycarbonates and polystyrenes, hard resins such as polyolefins including polypropylene and polyethylene, as well as metals and ceramics, for example, may be used. More preferred examples are thermoplastic resins such as polycarbonates and polystyrenes, and polyolefins such as polypropylene and polyethylene.

In order to reduce the flexing of the mesh portion of the filter, a reinforcement can also be provided on its surface (whether it is one or both surfaces of the mesh portion). In this case, the reinforcement may be formed of materials other than that used in the ring portion and resins or metals, for example, as well as composites of these can be used without particular limitations.

The ring portion is molded as an integral part of the mesh portion and even in the case where the elastic soft material is used only in the surface of the ring portion, the elastic soft resin and the hard material form an integral molding. If a reinforcement is provided on the surface of the mesh portion, it is preferably molded as an integral part of the ring portion or the mesh portion and, more preferably, the ring portion, the mesh portion and the reinforcement form an integral molding.

The adsorber of the invention has the elastic soft resin in the ring portion of each filter so that no liquid will leak out of the clearance between the filter and the housing or between the filter and the liquid port. Specifically, the filter made of the elastic soft resin is fixed pressed between the inner surface of each liquid port and an end face of the housing. Therefore, no extra packings need be used and still no liquid will leak into the clearance between the filter and the liquid port even if there are flaws or molding asperities on the end faces of the housing, the inner surfaces of the liquid ports, or even on their surfaces that contact the filters. Further, the ring portion of each filter is a principal seal member that creates a liquid-tight state between the liquid port and the housing and it proves effective even if the fusion between the liquid port and the housing is inadequate.

We now describe specifically how the housing is fixed to the liquid ports. Since the liquid ports 4 and 5 have the same shape irrespective of whether they are on the entrance side or on the exit side, the two liquid ports are not distinguished from each other in the following description.

The adsorber has an annular recess in the periphery of the inner surface of each liquid port. As shown in Fig. 3, the lateral surfaces of the annular recess in the liquid port are defined by the outer lateral surface of an annular rib provided inside the liquid port, the inner lateral surfaces of the shoulder of the liquid port and its bottom surface. The filter is mounted on the liquid port by fitting an end of its ring portion into this annular recess. Thus, the annular recess formed in the periphery of the inner surface of the liquid port prevents the annular elastic member from being displaced after fitting of the filter.

As shown in Fig. 3, the other end of the ring portion of the filter projects from the annular recess so that the ring portion of the filter contacts the inner surface of the liquid port at one end (the end portion of the side which contacts the liquid port) and contacts the end face of the housing at the other end (the end portion of the side which contacts the housing). In other words, the liquid port is fixed in the housing with the ring portion of the filter being pressed between the inner surface of the liquid port and an end face of the housing. With this design, even if an end face of the housing, the inner surface of the liquid port and even their surfaces that contact the ring portion of the filter have flaws and molding asperities or even if small foreign matter is lodged between an end face of the housing and the ring portion of the filter or between the inner surface of the liquid port and the ring portion of the filter, the liquid can be prevented from leaking to the lateral surface of the ring portion of the filter. The cross-sectional shape of the ring portion of the filter is not limited to the rectangle shown in Fig. 3 and it may be of any other shapes including circular, semicircular, elliptic, generally elliptic, triangular and trapezoidal shapes.

The annular rib on the liquid port portion is preferably provided in order to ensure that the present invention brings about the intended advantages but the invention works effectively in the absence of such annular rib.

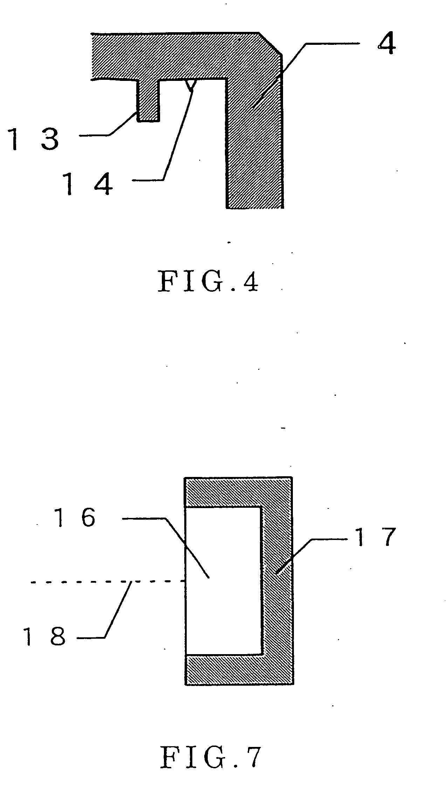

A method of making the invention more effective is by providing a slightly projecting ridge (see Fig. 4) on the bottom of the annular recess and on one or both end faces of the housing so that the force pressing the ring portion is sufficiently concentrated to ensure more positive prevention of liquid leakage.

We now describe a specific example of the preferred embodiment in which the filter of the invention is applied to an adsorber having an internal capacity of 100 - 1000 mL. The ring portion of the filter has an inside diameter which is approximately equal to or slightly larger than the inside diameter of each end face of the housing and it has an outside diameter which is approximately equal to or smaller than the outside diameter of each end face of the housing. The housing can use any shapes and dimensions without particular limitations if they fit the characteristics of the resin used and commonly used dimensions are 50 - 300 mm for the drum length, 40 - 100 mm for the inside diameter, and 2 - 5 mm for the wall thickness of end faces. As for the ring portion, the width is about 2 - 5 mm and the height is about 3 - 10 mm. The mesh portion is fitted in a position which is almost the center of the ring portion.

In order to fix the filter in a liquid-tight manner between the inner surface of the liquid port and each end face of the housing, the ring portion of the filter has to be pressed between these areas. The length by which the ring portion is pressed can be determined without particular limitations if it suits the characteristics and hardness of the resin used; compared to the case where the ring portion is not pressed, the length of pressing is usually about 0.1 - 0.5 mm.

As a result of this liquid-tight fixing of the liquid ports on the housing, a liquid outflow or inflow compartment is formed between the inner surface of the liquid port and the mesh portion of the filter. In the embodiment under consideration, the filter is molded separately from the liquid port and thereafter fitted on the inner surface of the liquid port. If desired, the two members may be molded as an integral unit so that the filter is fixed more positively to the liquid port to ensure more effective prevention of liquid leakage.

To describe a specific way to make an integral molding, the mesh and ring portions of the filter are first injection molded en masse to make a shaped filter which is then inserted into a liquid port forming die, where a liquid port forming resin is injection molded; this process is commonly called "insert molding".

If the adsorber under consideration is to be used in medical applications, namely, if it is a medical adsorber, sterilization is effected prior to use. Sterilization can be performed by known methods including treatment with ethylene oxide gas and autoclaving. In autoclaving, the interior of the adsorber (the adsorbent compartment and the liquid compartment) is filled with a liquid innocuous to the human body (e.g. physiological saline or sterile water) and the open areas (the liquid inlet and the liquid outlet) are sealed with an elastic member.

### Industrial Applicability of the Invention

The medical adsorber of the invention comprises a housing, an adsorbing unit packed within said housing, a liquid entrance port and a liquid exit port that are provided at opposite ends of said housing, and a filling spillage preventing filter for packing and retaining said adsorbing unit within said housing; the adsorber is characterized in that said liquid ports are fixed in the housing by pressing the ring portion of said filling spillage preventing filter between the inner surface of each of said liquid entrance and exit ports and an end face of the housing. With this design, even if the end faces of the housing, the inner surfaces of the liquid filling spillage preventing ports and even their surfaces that contact the filling spillage preventing filter have flaws and molding asperities or even if small foreign matter is lodged on the surfaces that contact the filling spillage preventing filter, no liquid will leak into the clearance between each of the liquid entrance and exit ports and the filling spillage preventing filter.

Hence, according to the present invention, there is provided an adsorber comprising a housing, filters fitted into liquid ports, and an adsorbing unit packed between the filters and the adsorber is characterized in that the number of its constituent parts is not increased but the pathways and gaps through which the liquid will seep to the lateral sides of the filters are eliminated to ensure that the liquid will not leak into the clearance between each filter and a lateral side of the liquid port.

## Claims

1. Adsorber, comprising a housing (2), an adsorbing unit (3) packed in said housing, a liquid port (4) on the entrance side which is fitted in an end portion of said housing and which has a liquid inlet (6), a liquid port (5) on the exit side which is fitted in the other end portion of said housing and which has a liquid outlet (7), and a filling spillage preventing filter that has a mesh portion which comprises
- a fabric mesh portion (8; 9) and an integral ring portion (10; 11) comprising an elastic soft resin for reinforcing the periphery of said mesh portion,
or
- a fabric mesh portion (8;9;18) and an integral ring portion (10;11;12) which is an integral molding (1 6) of a hard material for reinforcing the periphery of said mesh portion (18) and an elastic soft resin (1 7) covering the surface of said hard material,
the elastic soft resin being a resin selected from among silicone rubbers, polyurethane rubbers, isoprene rubbers, vulcanized rubbers, butadiene rubbers, ethylene-propylene rubbers, polyolefinic elastomers and styrenic elastomers,
the ring portion (10;11;12) being fixed to said housing (2) as it is pressed between the inner surface of each liquid port (4;5) and each end face of the housing.

2. Adsorber according to claim 1, **characterized in that** the hard material has a higher material hardness than the elastic soft resin formed on the surface of said hard material.

3. Adsorber according to claim 1 or 2, **characterized by** having a reinforcement of the mesh portion on the surface of the fabric mesh portion.

4. Adsorber according to any one of claims 1 to 3, **characterized in that** the mesh portion (8;9) and the ring portion (10;11) form an integral molding.

5. Adsorber according to any one of claims 1 to 3, **characterized in that** the mesh portion (8;9), the ring portion (10;11;12) and the reinforcement form an integral molding.

6. Use of the adsorber as claimed in any one of claims 1 to 5 as medical adsorber in which the liquid is a body fluid.

## Patentansprüche

1. Adsorber mit einem Gehäuse (2), einer in diesem Gehäuse untergebrachten Adsorbereinheit (3), einer Flüssigkeitsöffnung (4) auf der Eingangsseite, welche in einen Endbereich des Gehäuses eingefügt ist und welche einen Flüssigkeitseinlass (6) aufweist, einer Flüssigkeitsöffnung (5) auf der Ausgangsseite, welche in den anderen Endbereich des Gehäuses eingefügt ist und welche einen Flüssigkeitsauslass (7) aufweist, und einem Filter zur Verhinderung von Füllüberlauf, welcher einen Siebbereich besitzt, der aufweist:
- einen Maschengewebebereich (8; 9) und einen integrierten Ringbereich (10; 11) mit einem elastischen Weichharz zur Verstärkung des Umfangs dieses Maschenbereiches,
oder
- einen Maschengewebebereich (8; 9; 18) und einen integrierten Ringbereich (10; 11; 12), der ein integriertes Pressteil (16) aus einem harten Material zur Verstärkung des Umfangs dieses Maschenbereiches (18) und einem elastischen Weichharz (17) ist, welches die Oberfläche des harten Materials abdeckt,
wobei das elastische Weichharz ein Harz ist, das aus Silicongummis, Polyurethangummis, Isoprengummis, Vulkanisaten, Butadiengummis, Ethylen-Propylen-Gummis, Polyolefin-Elastomeren und Styrol-Elastomeren ausgewählt ist,
wobei der Ringbereich (10; 11; 12) an dem Gehäuse (2) befestigt wird, wenn er zwischen die Innenfläche jeder Flüssigkeitsöffnung (4; 5) und jede Endseite des Gehäuses gepresst wird.

2. Adsorber gemäß Anspruch 1, **dadurch gekennzeichnet, dass** das harte Material einer höhere Materialhärte aufweist als das elastische Weichharz, das auf der Oberfläche dieses harten Materials ausgebildet ist.

3. Adsorber gemäß Anspruch 1 oder 2, **dadurch gekennzeichnet, dass** er eine Verstärkung des Siebbereiches auf der Oberfläche des Maschengewebebereiches aufweist.

4. Adsorber gemäß irgendeinem der Ansprüche 1 bis 3, **dadurch gekennzeichnet, dass** der Maschenbereich (8; 9) und der Ringbereich (10; 11) ein einstückiges Pressteil bilden.

5. Adsorber gemäß irgendeinem der Ansprüche 1 bis 3, **dadurch gekennzeichnet, dass** der Maschenbereich (8; 9), der Ringbereich (10; 11; 12) und die Verstärkung ein einstückiges Pressteil bilden.

6. Gebrauch des Adsorbers gemäß irgendeinem der Ansprüche 1 bis 5 als medizinischer Adsorber, in welchem die Flüssigkeit eine Körperflüssigkeit ist.

## Revendications

1. Adsorbeur comprenant un logement (2), une unité d'adsorption (3) qui garnit ledit logement, un orifice pour liquide (4) du côté entrée, qui est pratiqué dans une portion terminale dudit logement et qui possède une entrée pour liquide (6), un orifice pour liquide (5) du côté sortie, qui est pratiqué dans l'autre portion terminale dudit logement et qui possède une sortie pour liquide (7), et un filtre empêchant le déversement lors du remplissage, qui possède une portion à maille qui comprend :
- une portion en tissu à maille (8 ; 9) et une portion annulaire intégrante (10 ; 11) comprenant une résine molle élastique pour renforcer la périphérie de ladite portion à maille ;
ou
- une portion en tissu à maille (8 ; 9 ; 18) et une portion annulaire intégrante (10 ; 11; 12) qui représente une moulure intégrante (16) constituée d'une matière dure pour renforcer la périphérie de ladite portion à maille (18), une résine molle élastique (17) recouvrant la surface de ladite matière dure ;
la résine molle élastique représentant une résine choisie parmi le groupe comprenant des caoutchoucs de silicone, des caoutchoucs de polyuréthane, des caoutchoucs d'isoprène, des caoutchoucs vulcanisés, des caoutchoucs de butadiène, des caoutchoucs d'éthylène-propylène, des élastomères polyoléfiniques et des élastomères styréniques ;
la portion annulaire (10 ; 11 ; 12) étant fixée audit logement (2) lorsqu'elle est comprimée entre la surface interne de chaque orifice pour liquide (4 ; 5) et chaque face terminale du logement.

2. Adsorbeur selon la revendication 1, **caractérisé en ce que** la matière dure possède une dureté de matière supérieure à celle de la résine molle élastique formée à la surface de ladite matière dure.

3. Adsorbeur selon la revendication 1 ou 2, **caractérisée par** le fait de posséder un renforcement de la portion à maille à la surface de la portion de tissu à maille.

4. Adsorbeur selon l'une quelconque des revendications 1 à 3, **caractérisé en ce que** la portion à maille (8 ; 9) et la portion annulaire (10 ; 11) forment un produit moulé en une seule pièce.

5. Adsorbeur selon l'une quelconque des revendications 1 à 3, **caractérisé en ce que** la portion à maille (8 ; 9), la portion annulaire (10 ; 11 ; 12) et le renforcement forment un produit moulé en une seule pièce.

6. Utilisation de l'adsorbeur selon l'une quelconque des revendications 1 à 5, comme adsorbeur médical dans lequel le liquide est un fluide corporel.
